# EUROPEAN PATENT SPECIFICATION

(11) **EP 3 313 450 B1**
(45) Date of publication and mention of the grant of the patent: **15.03.2023**
(21) Application number: 16738081.5
(22) Date of filing: 23.06.2016
(51) Int. Cl.: A61K 47/32

(54) **NANOGEL COMPOUND**
NANOGELVERBINDUNG
COMPOSÉ DE NANOGEL

(30) Priority: 25.06.2015 DE 102015211821
(43) Date of publication of application: 02.05.2018
(73) Proprietor: Freie Universität Berlin, 14195 Berlin (DE)
(72) Inventor: CALDERÓN, Marcelo, 12209 Berlin (DE); MOLINA, Maria, 01187 Dresden (DE); WEDEPOHL, Stefanie, 10435 Berlin (DE)
(74) Representative: Maikowski & Ninnemann Patentanwälte Partnerschaft mbB
(86) International application number: PCT/EP2016/064567
(87) International publication number: WO 2016/207296

(56) References cited:
- US-A1- 2004 001 892
- Jim Moselhy ET AL: "Characterization of complexation of poly (N-isopropylacrylamide-co-2-(dimethylamino ) ethyl methacrylate) thermoresponsive cationic nanogels with salmon sperm DNA", International journal of nanomedicine, 1 January 2009 (2009-01-01), pages 153-164, XP055307980, New Zealand Retrieved from the Internet: URL:https://www.dovepress.com/getfile.php? fileID=5198
- JULIO C. CUGGINO ET AL: "Thermosensitive nanogels based on dendritic polyglycerol and N-isopropylacrylamide for biomedical applications", SOFT MATTER, vol. 7, no. 23, 1 January 2011 (2011-01-01), page 11259, XP055308003, GB ISSN: 1744-683X, DOI: 10.1039/c1sm06357j

## Description

The instant invention relates to a nanogel compound according to the preamble of claim 1 , to a medicament comprising such a nanogel compound according to the preamble of claim 6 and to a method for producing such a nanogel compound according to the preamble of claim 8.

This nanogel compound can be used, e.g., in the field of nanotherapeutics, such as anti-cancer nanotherapeutics.

Within the area of anticancer nanotherapeutics, it is anticipated that nanotechnology based formulations will enhance the efficiency of the free form of drugs by imparting targeting to the desired organ/tissue/cell/cellular compartment and, concomitantly, reducing the systemic toxicity of the drug.

Within the last decade, significant efforts have been made in the development of stimuli-responsive drug delivery systems.' The use of stimuli-responsive nanocarriers offers an interesting opportunity for drug and gene delivery where the delivery system becomes an active participant, rather than passive vehicle, in the optimization of therapy. The benefit of these "smart" nanocarriers is especially important when the stimuli are unique to disease pathology, allowing the nanocarrier to respond specifically to the pathological "triggers". Selected examples of biological stimuli that can be exploited for targeted drug delivery systems include pH, temperature, redox microenvironment, etc.²

Among different stimuli-sensitive delivery modalities, temperature-sensitive drug delivery systems offer great potential over their counterparts due to their versatility in design, tunability of phase transition temperatures, passive targeting ability, and in situ phase transitions. Thus, thermosensitive drug delivery systems can overcome many of the hurdles of conventional drug delivery systems in order to increase drug efficacies, drug targeting, and decrease drug toxicities.

Additionally, near-infrared (NIR) radiation has been used often to externally trigger structural changes in thermoresponsive materials embedded with metal nanoparticles or conjugated polymers.³ NIR radiation has proven to be a promising tool for both *in vivo* imaging and photothermal cancer treatment. A key advantage of using light in the NIR window, ca. 650-900 nm, is its minimal absorbance by skin and tissues. This window is limited at the lower end by the absorbance of hemoglobin and at the high end by the absorbance of water. Between these boundaries, light can penetrate tissue in the order of hundreds of micrometers to centimetres, enabling whole-body optical imaging.

Various types of nanoparticles have been designed and developed that exhibit high absorption in the NIR range and have been used for photothermal drug release either alone or as components within the polymer composites.⁴

Hyperthermia therapy using carbon nanotubes,⁵ magnetic nanomaterials,⁶ or gold nanoparticles⁷ activated with NIR has shown to be effective in the treatment of skin,⁸ breast,⁹ liver,¹⁰ and ovarian cancers.¹¹ However there are still biocompatibility issues regarding these particles that make them less attractive for *in vivo* applications.

Nanogels are crosslinked polymer networks which are able to encapsulate large quantities of water. Smart nanogels are those which respond to an external stimulus, such as pH, light, or temperature.¹² In particular thermoresponsive nanogels are mostly used as drug delivery systems due to the simplicity of the system to be trigger.

Poly N-isopropylacrylamide (PNIPAM) nanogels can be used as thermoresponsive drug delivery systems because PNIPAM exhibits a lower critical solution temperature (LCST) of approximately 32 °C. Therefore, the polymer can undergo temperature-induced reversible coilto-globule phase transitions, and this can influence release behaviors of drug entrapped in them.¹³ By combining PNIPAM with dendritic polyglycerol (dPG), Calderón et al. developed a thermoresponsive nanogel which provide sizes in the biomedical relevant range between 50 and 200 nm.¹⁴ US 2004/0001892 A1 refers to a semi-interpenetrating polymer network comprising a cross-linked thermo-responsive polymer (e.g. hydrogels made of isopropyl acrylamide) and a linear polymer entangled with the thermo-responsive polymer wherein the linear polymer is derivatized with a bioactive molecule. In JULIO C. CUGGINO ET AL: "Thermosensitive nanogels based on dendritic polyglycerol and N-isopropylacrylamide for biomedical applications", SOFT MATTER, vol. 7, no. 23, 1 January 2011 (2011-01-01), page 11259, nanogels based on dendritic polyglycerol and N-isopropylacrylamide for biomedical applications are disclosed.

In order to modify the properties of PNIPAM nanogels, different copolymers¹⁵ and composites^{3b, 6, 16} were already studied and resulted in changes in the swelling behavior, LCST, release rate, and multiresponse.

It is an object of the instant invention to provide new biocompatible compounds that can be used the field of nanotherapeutics.

This object is achieved by a nanogel compounds having the features specified in claim 1. Such a nanogel compound comprises a nanogel matrix component comprising a dendritic polyglycerol crosslinked by poly(N-isopropylacrylamide).

It comprises 2-acrylamido-2-methyl-1-propanesulfonic acid as a first conjugation component which forms an interpenetrating or semi-interpenetrating network with the nanogel matrix component. Thus, the nanogel compound consists of the nanogel matrix component and the first conjugation component.

In accordance with IUPAC definitions, the term "interpenetrating network" is to be understood as a polymer comprising two or more networks that are at least partially interlaced on a molecular scale but not covalently bonded to each other and cannot be separated unless chemical bonds are broken. Similarly, the term "semi-interpenetrating network" is to be understood as a polymer comprising one or more polymer networks and one or more linear or branched polymers characterized by the penetration on a molecular scale of at least one of the networks by at least some of the linear or branched macromolecules. A semi-interpenetrating network is distinguished from an interpenetrating network because the constituent linear or branched macromolecules can, in principle, be separated from the constituent polymer network(s) without breaking chemical bonds; it is a polymer blend. The preceding definitions are taken from the following source: IUPAC. Compendium of Chemical Terminology, 2nd ed. (the "Gold Book"). Compiled by A. D. McNaught and A. Wilkinson. Blackwell Scientific Publications, Oxford (1997). XML on-line corrected version: http://goldbook.iupac.org (2006-) created by M. Nic, J. Jirat, B. Kosata; updates compiled by A. Jenkins. ISBN 0-9678550-9-8. doi:10.1351/goldbook.

It was surprisingly found that such a charged first conjugation component is very well suited to interact with pharmaceutically active substances. In addition, upon shrinkage of the nanogel matrix component in a nanogel compound according to the instantly claimed invention, one would expect that the charged first conjugation component shrinks together with the nanogel matrix component. Surprisingly, the inventors of the instant invention have found that this is not the case. Rather, the charged first conjugation component is exposed to the outside of the nanogel matrix component if the latter shrinks (i.e. if its hydrodynamic diameter is reduced). Due to the exposure of the charged first conjugation component to the outside of the nanogel matrix component, the interaction between the first charged conjugation component and a drug loaded into the nanogel compound is destabilized. As a consequence, the drug can be released from the nanogel compound.

A reduction of the hydrodynamic diameter of the nanogel matrix components can be achieved by heating the nanogel compound to a temperature above the transition temperature of the nanogel matrix component.

Heating the nanogel matrix component above its transition temperature leads to a collapse of the nanogel matrix component. However, still then the interpenetrating or semi-interpenetrating first conjugation component remains part of the nanogel compound. Thus, the electrostatic interaction between the charged first conjugation component and a drug loaded into the nanogel compound is reduced, but still exists. Consequently, the release of the drug is still subject to electrostatic interactions of the drug with the first conjugation component. This leads to a controlled (retarded, sustained) release of the drug from the nanogel compound.

Further suited acids to be used as first conjugation component are acrylic acid, methacrylic acid, , and 4-acryloylamine-4-(carboxyethyl) heptanodioic acid.

A suited methacrylate to be used as a further first conjugation component is a di(C₁-C₁₀)alkylamino(C₁-C₁₀)alkyl methacrylate. Thereby, C₁, C₂, C₃, C₄, C₅, C₆, C₇, C₈, C₉ or C₁₀ alkyls are possible in any position of this methacrylate independently from each other.

Specific examples of well suited methacrylates are dimethylaminomethyl methacrylate, dimethylaminoethyl methacrylate and dimethylaminopropyl methacrylate.

Suited methacrylamides are N-(3-((4-((3-aminopropyl)amino)butyl)amino)propyl)methacrylamide, N-(3-((4-aminobutyl)amino)propyl)methacrylamide, N-(2-((2-aminoethyl)(methyl)amino)ethyl) methacrylamide, and N-(2- (bis(2-aminoethyl)amino)ethyl)methacrylamide.

Suited acrylamides are N-(3-((4- aminobutyl)amino)propyl)acrylamide, N-(2-((2-aminoethyl)(methyl)amino)ethyl)acrylamide, and N-(2-(bis(2-aminoethyl)amino)ethyl)acrylamide.

It should be noted that these components can be used in any desired combination as first conjugation component. Thus, it is possible that two or more substances build up the first conjugation component, wherein the substances need not to be covalently bound each other. Rather, a mixture of the above-mentioned substances can be used.

Alternatively, in an embodiment, the nanogel compound comprises a second conjugation component which forms an interpenetrating or semi-interpenetrating network with the nanogel matrix component, wherein the second conjugation component is chosen from the group consisting of polyaniline, polypyrrole, poly(N-(C₁-C₁₀)alkyl aniline), poly(3,4-ethylenedioxythiophene), 2-ethylhexylcyclopentadithiophene-co-2,1,3-benzothiadiazole, 2-ethylhexylcyclopentadithiophene-co-2,1,3-benzoselenadiazole, and combinations thereof. Thereby, an C₁, C₂, C₃, C₄, C₅, C₆, C₇, C₈, C₉ or C₁₀ alkyl is possible within the poly(N-(C₁-C₁₀)alkyl aniline.

In an embodiment, the nanogel compound consists of a) the nanogel matrix component and b) the first conjugation component and/or the second conjugation component.

In an embodiment, the poly(N-(C₁-C₁₀)alkyl aniline) is poly(N-methyl aniline).

In an embodiment, the second conjugation component is polyaniline (PANI) or polypyrrole (PPY). These conjugated polymers turned out to be very good reaction partners for the nanogel matrix component for forming a semi-interpenetrating network.

It is also possible that the nanogel compound does not comprise a first conjugation component, but only the nanogel matrix component according to the above-given explanations and a second conjugation component according to the above-given explanations. Thus, in this embodiment, the nanogel compound does not make use of a charged first conjugation component, but only of the polymeric second conjugation component. This embodiment is an individually claimed part of the instant disclosure. It can be combined in any way with any other embodiments of the instantly described invention.

According to the invention, the nanogel matrix component comprises a polyglycerol and another substance that cross-links individual polyglycerol units with each other. This other substance is, in an embodiment, a thermoresponsive polymer so as to introduce thermoresponsive properties into the nanogel matrix component. Suited thermoresponsive polymers are listed above as possible components for the nanogel matrix component. In addition, the polyglycerol is a dendritic (hyperbranched) polyglycerol. Furthermore, a functionalization of the polyglycerol is possible. To give an example, vinyl-functionalized polyglycerol can be used as one of the matrix components. According to the invention a dendritic polyglycerol crosslinked by poly(N-isopropylacrylamide) is used as nanogel matrix component. The dendritic polyglycerol can be functionalized by, e.g., vinyl groups to allow for cross-linking in a particularly suited manner.

In an embodiment, the first conjugation component is dimethylaminoethyl methacrylate (DMAEM) or according to the invention 2-acrylamido-2-methyl-1-propanesulfonic acid (AMPS). These two first conjugation components can be (either alone or in combination) very well combined with a nanogel matrix component as explained in the preceding paragraph, e.g., comprising or consisting of a dendritic polyglycerol crosslinked by poly(N-isopropylacrylamide).

The description also relates to the medical indication of a nanogel compound according to the preceding explanations, namely for the use of such a nanogel compound as carrier for drug delivery to a human or animal in need thereof. Thereby, the nanogel compound can be combined with any drug that is able to undergo electrostatic or other interactions with the first conjugation component and/or the second conjugation component. As explained above, the nanogel compound exhibits sustained-release properties that can be exploited in many medical applications in a particularly suited way. To give an example, it was found that the anti-cancer drug doxorubicin (DOXO) electrostatically strongly interacts with the first conjugation component, AMPS. Upon heating the nanogel compound above the transition temperature of the nanogel matrix component, a low but constant DOXO release is achieved over an extended period of time. This comparatively low DOXO release results in a low DOXO concentration inside the cancer cell which is not detected by the efflux pumps otherwise clearing DOXO from the resistant cancer cell. Thus, the instantly described nanogel compound offers the possibility to deliver drugs in a very specific way that allows medical treatments that are otherwise (i.e. upon delivery of a higher concentration of specific drugs to a cell) not possible at all.

The instantly claimed invention also relates to a medicament comprising a nanogel compound according to the preceding explanations and a pharmaceutically active substance bound to the nanogel compound. Thereby, the pharmaceutically active substance can be bound in a covalent or in a non-covalent manner to the nanogel compound. Particularly, a non-covalent interaction between the nanogel compound and the pharmaceutically active substance is established. The non-covalent interaction can be an electrostatic interaction. In such a case, a charged pharmaceutically active substance or a charged form of a pharmaceutically active substance is particularly suited.

In an embodiment, the pharmaceutically active substance is an anti-cancer drug, such as doxorubicin, paclitaxel, methotrexate, cisplatinum, 5-fluorouracil and combinations thereof. The invention further relates to a method for producing a nanogel compound according to claim 8 providing a nanogel matrix component comprising dendritic polyglycerol crosslinked by poly(N-isopropylacrylamide).

The nanogel matrix components can, e.g., be produced by precipitation polymerization¹⁴, thermonanoprecipitation,¹⁷ or miniemulsion.¹⁸

In a further method step, an in situ radical polymerization of a first conjugation component in presence of the nanogel matrix component is performed. Thereby, the first conjugation component is a charged component chosen from the group consisting of 2-acrylamido-2-methyl-1-propanesulfonic acid.

Furthermore, the invention relates to the claimed nanogel compound for use in a method of drug delivery by means of a drug comprising a nanogel compound according to the preceding explanations and a pharmaceutically active substance, wherein the drug release from the nanogel compound is triggered by NIR. Speaking more generally, the instant invention relates to the claimed nanogel compound for use in methods of controlled delivery and release of pharmaceutically active substances from a nanogel compound according to the preceding explanations, in particular by means of thermal effects or by means of changing the pH value. All embodiments explained with respect to the nanogel compound can be combined in any desired weight. In addition, they can be transferred to the described or claimed use of the nanogel compounds, to the described or claimed medicament, and to the described or claimed methods. In addition, all embodiments of the use of the nanogel compound, the medicament and the methods can be transferred to the nanogel compound itself, the use, the medicament and the methods in any desired way and combination.

Further aspects and details of the instant invention will be explained with respect to Figures and exemplary embodiments. Only embodiments exemplified and disclosed in the claims are part of the invention. In the Figures:
- Figure 1: shows a schematic depiction of the structural behavior of an exemplary embodiment of a nanogel compound,
- Figure 2A: shows the influence of the pH value on the zeta potential and the size of PNIPAM-dPG/DMAEM,
- Figure 2B: shows the influence of the temperature on the zeta potential of PNIPAM-dPG/DMAEM,
- Figure 2C: shows the influence of the pH value on the zeta potential and the size of PNIPAM-dPG/AMPS,
- Figure 2D: shows the influence of the temperature on the zeta potential of PNIPAM-dPG/AMPS,
- Figure 3: shows the cumulative release of DOXO from PNIPAM-dPG/DMAEM and PNIPAM-dPG/AMPS at 25 and 42 °C and at pH 7.4,
- Figure 4: shows a plot of the cytotoxicity profile of PNIPAM-dPG/DMAEM and PNIPAM-dPG/AMPS determined by an MTT assay,
- Figure 5A: shows a plot of the cytotoxicity profile of DOXO-loaded PNIPAM-dPG/DMAEM and PNIPAM-dPG/AMPS and free DOXO determined by an MTT assay in HeLa cells,
- Figure 5B: shows a plot of the cytotoxicity profile of DOXO-loaded PNIPAM-dPG/DMAEM and PNIPAM-dPG/AMPS and free DOXO determined by an MTT assay in resistant cell lines (KB-V1),
- Figure 6: shows the *in vivo* evaluation of bare and DOXO-loaded PNIPAM-dPG, PNIPAM-dPG/AMPS, PNIPAM-dPG/DMAEM, and of free DOXO,
- Figure 7: shows a schematic depiction of the synthesize and of the structural behavior of a nanogel compound not comprising a charged conjugation component,
- Figure 8A: shows the thermoresponsive behavior of PNIPAM-dPG/PANI measured by Dynamic Light Scattering (DLS),
- Figure 8B: shows a micrograph obtained by Atomic Force Microscopy (AFM) of PNIPAM-dPG/PANI,
- Figure 8C: shows a micrograph obtained by Atomic Force Microscopy (AFM) of PNIPAM-dPG/PPY,
- Figure 9: shows the change of temperature over time of irradiation for dispersions of PNIPAM-dPG/PANI at different concentrations,
- Figure 10A: shows the cytotoxicity of PNIPAM and PNIPAM-dPG/PANI on A2780 cells as determined by an MTT assay,
- Figure 10B: shows the PNIPAM-dPG/PANI concentration in culture medium of A2780 cells as determined by absorbance measurement over time,
- Figure 11: shows the relative viabilities of A2780 cells, treated with PNIPAM-dPG/PANI or untreated, irradiated with NIR laser or heated to 42 °C,
- Figure 12: shows the tumor growth inhibition (%) of tumors in female nude mice treated with PBS, PNIPAM-dPG, PNIPAM-dPG/PANI, PNIPAM-dPG/PPY with and without irradiation,
- Figure 13: shows the cumulative release of DOXO from PNIPAM-dPG/PANI at pH 7.4 with and without NIR irradiation,
- Figure 14: shows the temperature change of a cell cluster of 1 million cells, incubated with drug-loaded semi interpenetrated nanogels, during irradiation with NIR laser,
- Figure 15: shows the cell viability of irradiated or non-irradiated DOXO-loaded PNIPAM-dPG/PANI or PNIPAM-dPG/PPY 24h after treatment and

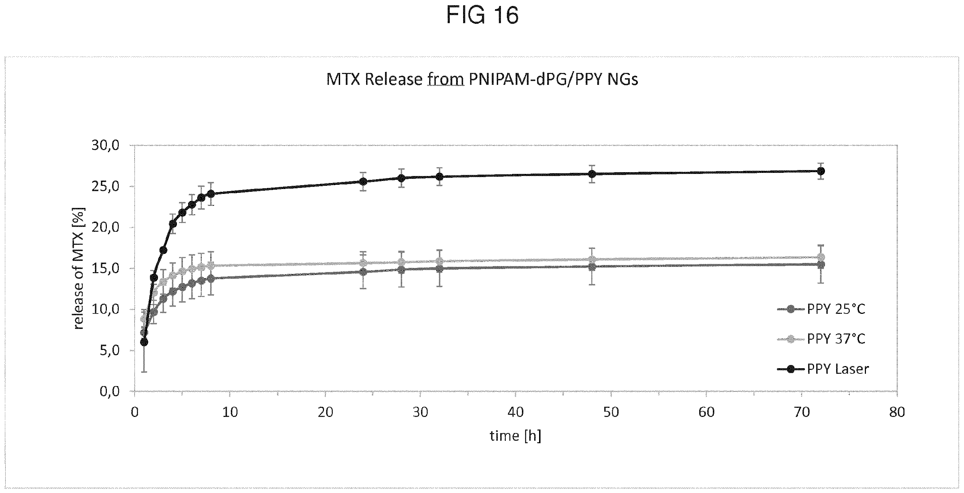

- Figure 16: shows MTX release from PNIPAM-dPG/PPY nanogels above or below the transition temperature, or after NIR laser irradiation.

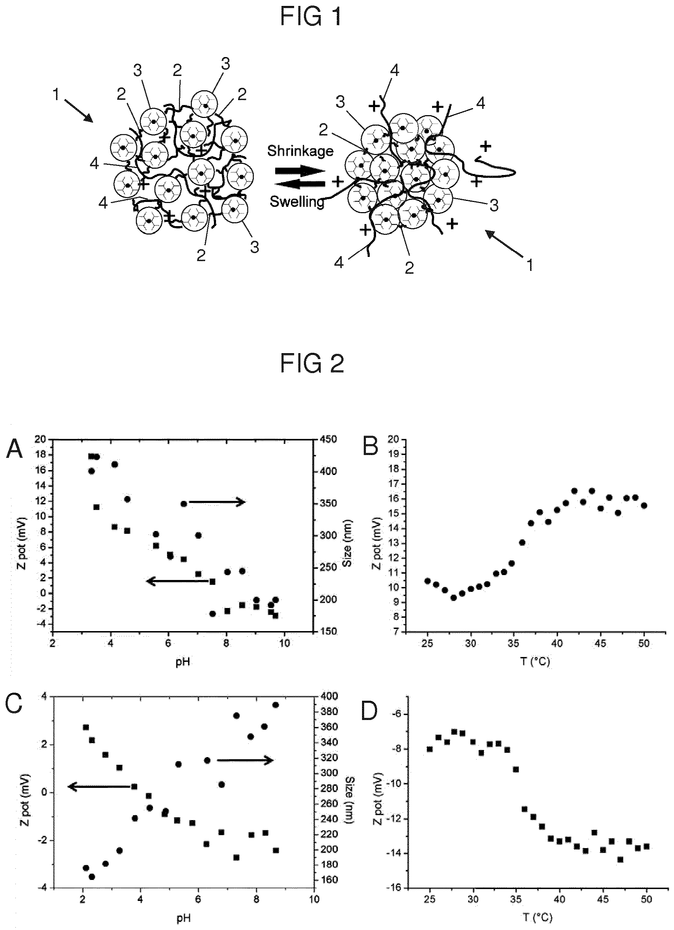

Figure 1 shows the structural behavior of a thermoresponsive nanogel compound 1 based on poly(N-isopropylacrylamide) (PNIPAM) 2 and dendritic polyglycerol (dPG) 3 which is semi interpenetrated (s-IPN) with a charged polymer, namely, dimethylaminoethyl methacrylate (DMAEM) 4 as first conjugation component. Alternatively, 2-acrylamido-2-methyl-1-propanesulfonic acid (AMPS) or another charged polymer could be used as first conjugation component. For sake of clarity, only some of the respective molecules of the individual components in the depiction of Figure 1 bear the corresponding numeral reference.

Upon a temperature increase, the nanogel compound 1 shrinks, i.e. its size is reduced. Thereby, the positive charges of DMAEM 4 are exposed to the outside of the core of the nanogel compound 1. If AMPS was used as first conjugation component, its negative charges would be exposed to the outside of the core of the nanogel compound. Thus, the temperature increase triggers an exposure of the charge of the first conjugation component.

The charge density of the nanogel compound can be controlled by the ratio between charged monomer (first conjugation component) and the nanogel matrix component as well as by the time of polymerization of the charged monomer. The successful semi-interpenetration of DMAEM or AMPS was proven by Fourier transform infrared spectroscopy (FTIR) and proton nuclear magnetic resonance (¹H NMR).

After the semi-interpenetration of the nanogel matrix component by the first conjugation component, the zeta potential of the network changes. When the nanogel matrix component is semi-interpenetrated by a cationic or anionic compound, the zeta potential is more positive or negative, respectively. By increasing the percentage of the charged compound in the network or the time of reaction of the charged compound, the zeta potential increases as well.

Interestingly, the transition temperatures of the nanogel compounds do not change after the semi-interpenetration with the first conjugation component. This behavior is opposite to the one observed when the first conjugation component is co-polymerized into the network of the nanogel matrix component .¹⁹

Two different behaviors can be obtained depending on the chosen first conjugation component. As a general rule, the size of the nanogel compound increases with the charge of the network. Thus, when increasing the pH, the positively charged PNIPAM-dPG/DMAEM nanogel compound shows a decrease of size. To the contrary, the negatively charged PNIPAM-dPG/AMPS nanogel compound shows an increase in size when the pH value is increased. This is shown in Figures 2A (PNIPAM-dPG/DMAEM) and 2C (PNIPAM-dPG/AMPS). Thereby, the circles in figures 2A and 2C relate to the size (right y-axis), whereas the squares relate to the zeta potential (left y-axis).

In the case of PNIPAM-dPG/DMAEM NGs the size decreased from -430 nm at pH 3 to -175 nm at pH 10 with a change in zeta potential from 20 to -2. The zeta potential of PNIPAM-dPG/AMPS NGs changed from 3 to -3 with the increase of pH from 2 to 10, followed by an increase of size from 150 nm to 400 nm. Moreover, after collapse of the nanogel compound, the zeta potential of the network increased. This is shown in Figures 2B (PNIPAM-dPG/DMAEM) and 2D (PNIPAM-dPG/AMPS). This can be explained by the exposition of the charged first conjugation component during the collapse of the thermoresponsive nanogel compound. This behavior can be exploited in different fields and applications, for example to encapsulate or release a cargo on demand.

In order to use these new nanogel compounds in drug delivery applications, the encapsulation of the anti-cancer drug doxorubicin (DOXO) was studied. The results of the encapsulation study are shown in Table 1. PNIPAM-dPG/AMPS encapsulates the double amount of DOXO compared to PNIPAM-dPG/DMAEM due to the electrostatic interaction. As a comparison PNIPAM/dPG without charges was also studied.

**Table 1. Loading capacity (L.C., in %) and loading efficiency (L.E., in %) of DOXO in PNIPAM, PNIPAM-dPG/AMPS, and PNIPAM-dPG/DMAEM.**

| Nanogel compound | %L.C. | %L.E. |
|---|---|---|
| PNIPAM/dPG (control) | 6.48 | 13.25 |
| PNIPAM-dPG/AMPS | 8.97 | 17.50 |
| PNIPAM-dPG/DMAEM | 4.30 | 9.00 |

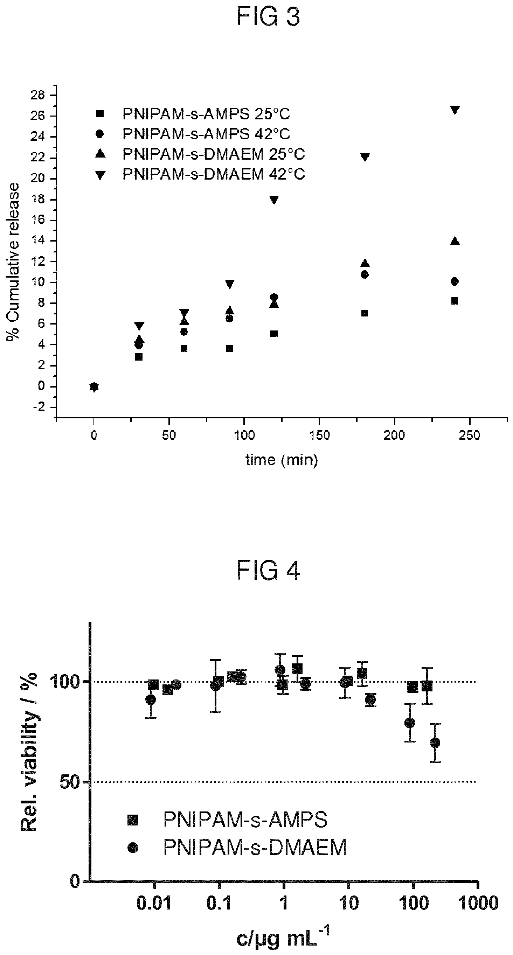

After the encapsulation, the release of DOXO was studied *in vitro.* For each nanogel compound, two experiments were performed below (25 °C) and above (42 °C) the transition temperature in order to see if the release is triggered by the collapse of the nanogel compound. The results are shown in Figure 3.

When an electrostatic interaction is present the release is slower than without electrostatic interaction, while for all nanogel compounds the release is faster at 42 °C than at 25 °C showing a temperature-dependent release.

The *in vitro* toxicity and cellular uptake in HeLa cell lines were studied for further utilization of these materials in biomedical applications. The results are shown in Figure 4. None of the tested nanogel compounds showed an effect on this cell line up to concentration of 1 mg mL¹, indicating their very good biocompatibility.

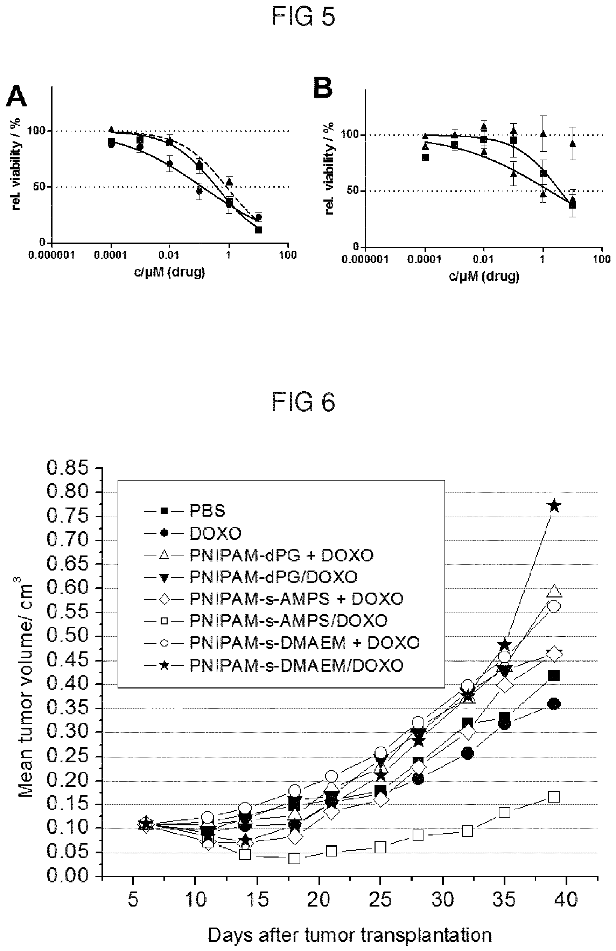

In order to use the nanogel compounds for anticancer therapy, the cytotoxicity of the DOXO-loaded nanogel compounds compared with free DOXO was studied in two different cell lines: sensitive-type HeLa cells and DOXO resistant cell line KB-V1. The results are shown in Figures 5A and 5B. Thereby, squares denote PNIPAM-dPG/AMPS, circles denote PNIPAM-dPG/DMAEM, and triangles denote free DOXO.

Briefly, with the sensitive-type HeLa cells (Figure 5A) the DOXO-dose dependent cytotoxicity curve of the nanogel compounds was similar to free DOXO. In contrast, with the cell resistant line (KB-V1) the external concentration of free DOXO of up to 10 µM had no effect on cytotoxicity, which is typical for resistant cell lines, indicating that the active internalization of DOXO is essential for toxicity (Figure 5B). The dose-response curve of the DOXO-loaded nanogel compounds resembled very closely that one obtained for the sensitive cells. This demonstrates the efficiency of these nanogel compounds as carriers for drugs to overcome drug resistance in tumor cell lines.

To further prove the efficacy of these carriers, *in vivo* tumor growth inhibition experiments were carried out. In order to test the potency of the DOXO-loaded nanogel compounds to overcome anthracycline resistance *in vivo,* nude mice were injected with HeLa-derived xervic carcinoma Adriamycin-resistant MaTu-ADR and treated twice with the DOXO-loaded nanogel compounds using phosphate-buffered saline (PBS) and bare (unloaded) nanogel compounds as negative control and compared with free DOXO.

The results are shown in Figure 6. It can be clearly seen that the group treated with DOXO-loaded PNIPAM-dPG/AMPS could effectively overcome the DOXO resistance of the cells and inhibited the tumor growth after 32 days of treatment. The efficiency of this carrier is likely due to the strong electrostatic interaction between the DOXO and the charges of the nanogel compound which enable the prolonged controlled release of the drug over the time, which provoked a low but constant DOXO concentration inside the cell which is not being detected by the efflux pumps of the cells.

In summary, novel drug carriers were developed by semi-interpenetration of a charged compound inside a thermoresponsive nanogel matrix component. These novel nanogel compounds increase the superficial charge on demand, after an increase in the temperature. The charge of the thermoresponsive nanogel compound can be finely tuned by the chosen charged component, the ratio of charged component to nanogel matrix component, and the polymerization time of the charged component inside the nanogel matrix component.

DOXO, an anti-cancer drug was encapsulated and efficiently released triggered by an increase in the temperature of the surrounding medium. While the encapsulation of the drug is increased due to the electrostatic interactions with the novel nanogel compound, the release is slower when this interaction is present. On the other hand, when electrostatic repulsion is observed, the release of the drug is much faster. Low cytotoxicity profile and good internalization of the nanogel compounds by tumor cells were shown. This is important for a use of the nanogel compounds as drug carrier. In comparison with free DOXO, the DOXO-loaded nanogel compounds have been proven to efficiently overcome the pump efflux of the drug resistant cells, showing a high cytotoxicity. More importantly, DOXO-loaded PNIPAM-dPG/AMPS has been proven to efficiently overcome the resistance of cells *in vivo.*

Thus, suited applications of the described nanogel compounds are controlled drug/protein delivery and anticancer therapy.

In further experiments, nanogel compounds comprising a semi-interpenetrating second conjugation component which is not a charged component have been prepared. No first conjugation component was used. These nanogel compounds are also very well suited as drug carriers, in particular for anticancer drugs.

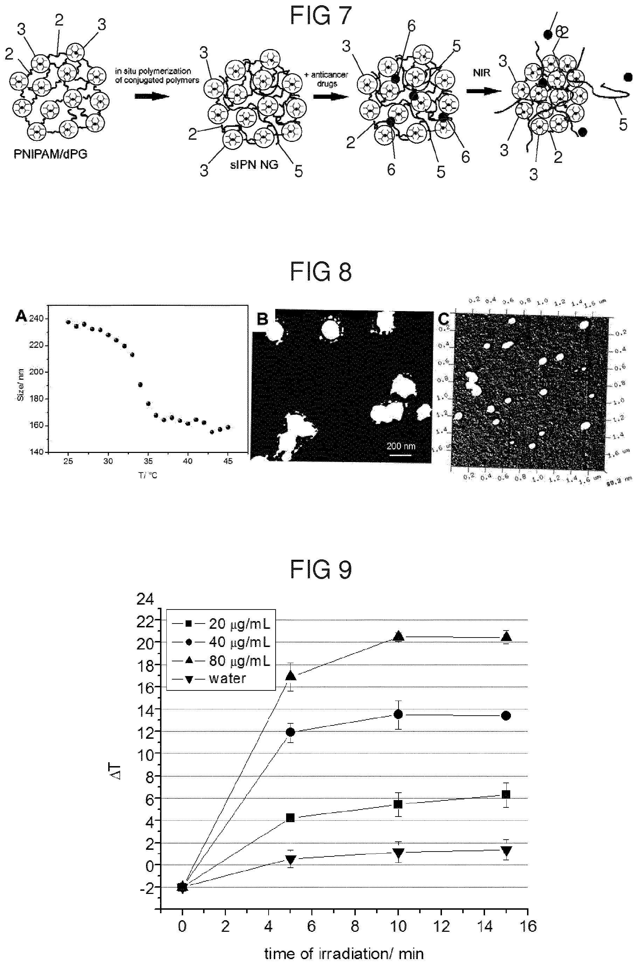

With reference to Figure 7, a thermoresponsive nanogel comprising a nanogel matrix 2, 3 which is semi-interpenetrated (s-IPN) with a conjugated polymer 5, namely, polyaniline (PANI) or polypyrrole (PPY) has been synthesized. This composite serves as nanogel compound. The conjugated polymer 5 (second conjugation component) generates heat after being irradiated by near infrared (NIR) radiation, inducing a local hyperthermia. This is also schematically depicted in Figure 7. The nanogel compound, because of being thermosensitive, undergoes a change in conformation that triggers different mechanisms like shrinkage of the nanogel compound, aggregation of the nanogel compound, drug 6 release from the nanogel compound (if it was previously loaded with this drug 6), uptake of the nanogel compound into a cell and/or a tissue as well as accumulation in a specific compartment, etc.

The thermoresponsive nanogel compound was synthesized based on the work of Cuggino et al.¹⁴ Poly(N-isopropylacrylamide) (PNIPAM) 2 was used as thermoresponsive polymer (a first nanogel matrix component) and vinyl functionalized dendritic polyglycerol (dPG) 3 was used as macro-crosslinker (a second nanogel matrix component). The synthesis of the nanogel compound (PNIPAM-dPG/PANI or PNIPAM-dPG/PPY) was done by an in situ redox polymerization of aniline or pyrrole in the presence of the thermoresponsive nanogel matrix component.

A stable PNIPAM-dPG/PANI or PNIPAM-dPG/PPY dispersion with reproducible size and thermoresponsive properties was obtained using 0.1 M of aniline or pyrrole and 30 min of polymerization. According micrographs obtained by atomic force microscopy are depicted in figures 8B and 8C. Figure 8A shows the thermoresponsive behavior of PNIPAM-dPG/PANI. Its transition temperature is at approximately 32 °C. Heating PNIPAM-dPG/PANI above its transition temperature leads to a significant decrease in size, i.e. to shrinkage or a collapse of PNIPAM-dPG/PANI.

A suited application of this nanogel compound is hyperthermia therapy. This was probed by studying the increase of the temperature of the nanogel compound under NIR radiation. As can be seen in Figure 9, the increase in temperature of a dispersion of the nanogel compound in a suited medium is dependent on the concentration of the nanogel compound. Thereby, the increase in temperature is proportional to the concentration of nanogel compound in the dispersion. When only water is irradiated, the temperature increases by 3 °C. If 20 µg mL⁻¹ of PNIPAM-dPG/PANI in water is irradiated under the same conditions, the temperature increases already by 8 °C, which is enough for mild hyperthermia conditions.²⁰

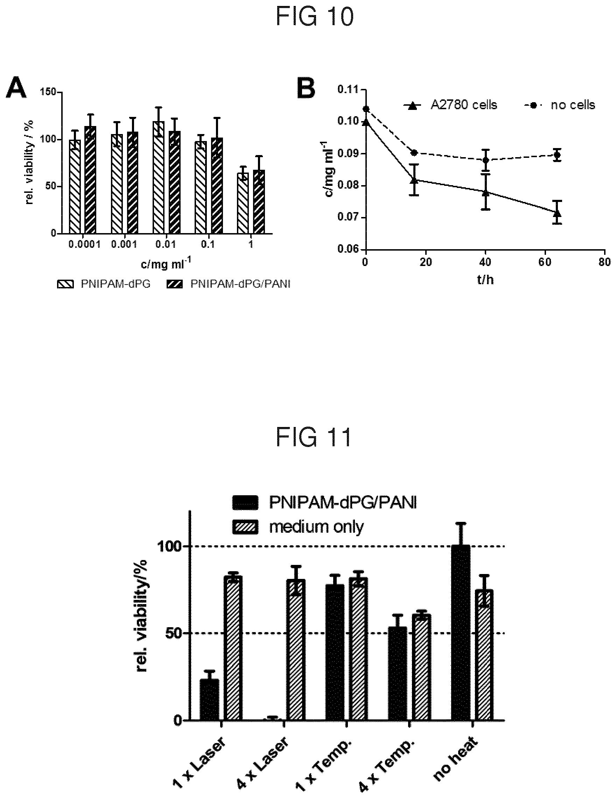

The cytotoxicity of the nanogel compound on A2780 ovarian cancer cells was studied by MTT assay (MTT stands for 3-(4,5-dimethylthiazol-2-yl)-2,5-diphenyltetrazolium bromide). The results depicted in Figures 10A and 10B.

The cells which were incubated with PNIPAM-dPG/PANI or PNIPAM-dPG alone, show high viability over 50 % up to a concentration of 1 mg mL⁻¹ (Figure 10A). Uptake of PNIPAM-dPG/PANI into cells was monitored indirectly by measuring the concentration in cell culture supernatant (Figure 10B). After 64 h, the concentration was about 0.03 mg mL⁻¹ less than initially incubated. Thus, this amount was taken up by the cells. Additionally, it was observed that the cells were dark colored after trypsinization and washing with PBS or media. The color remained after repeated trypsinization, reculturing, and washing. This confirms that the nanogel compound was located inside the cells.

For the study of the hyperthermia therapy *in vitro,* cells were released from a cell culture flask, washed and centrifuged into PCR tubes which allow for rapid transmission of heat through the walls of the tubes. In this way, the approximate temperature of the cell suspension or cell pellet could be monitored. The culture medium was removed leaving a small ball of approximately 1 million cells that had about the volume of a 5 µL drop. This cluster of cells, which may resemble a small tumor tissue, was irradiated with a NIR laser (785 nm) under constant monitoring of a thermal camera to ensure the temperature did not exceed 42 °C +/- 1 °C. As a control for the effect of hyperthermia alone, equally treated samples were incubated in a water bath at 42 °C. Irradiation or heating was performed one time for 5 min or three times for 5 min each with PNIPAM-dPG/PANI incubated cells as well as with untreated cells. Viabilities were measured after 24 h with MTT assay. The results are shown in Figure 11.

A2780 cells which had taken up PNIPAM-dPG/PANI heated up during irradiation with a NIR laser and showed a markedly reduced viability after a single treatment for 5 min, as compared to 80% viability of untreated control cells which were irradiated for the same time. Four irradiation cycles resulted in a complete loss of cell viability of PNIPAM-dPG/PANI treated cells, whereas the control cells again were not compromised. The effect of hyperthermia for 5 min alone was not measurable. Only a decrease in viability to about 60% could be observed if the hyperthermia is repeated four times. To control for the stress of centrifuging and standing as a cell cluster for a prolonged period of time outside of the incubator, another cell sample was treated in the same way but not heated or irradiated. These control cells showed no reduced viability, comparable to the untreated cells in the other series.

The cytotoxicity of the nanogel compound was also studied *in vivo* by two different essays. First a single treatment with doses from 10 to 100 mg kg⁻¹ showed that mice tolerated up to 100 mg kg⁻¹ of PNIPAM-dPG/PANI or PNIPAM-dPG/PPY without any significant sign of toxicity or drug-related body weight loss. In a second step, three mice were treated with the maximum tolerated dose (MTD) found in the single treatment, 100 mg kg⁻¹ once per day at 5 consecutive days. Mice were observed for a follow-up period of 18 days. No significant toxic effect was observed during that period. A dark blue color was observed in the tails, liver area, and blood vessel system in the abdomen.

Body weight was slightly reduced to 97.2% at day 11, but mice recovered very fast from this effect.

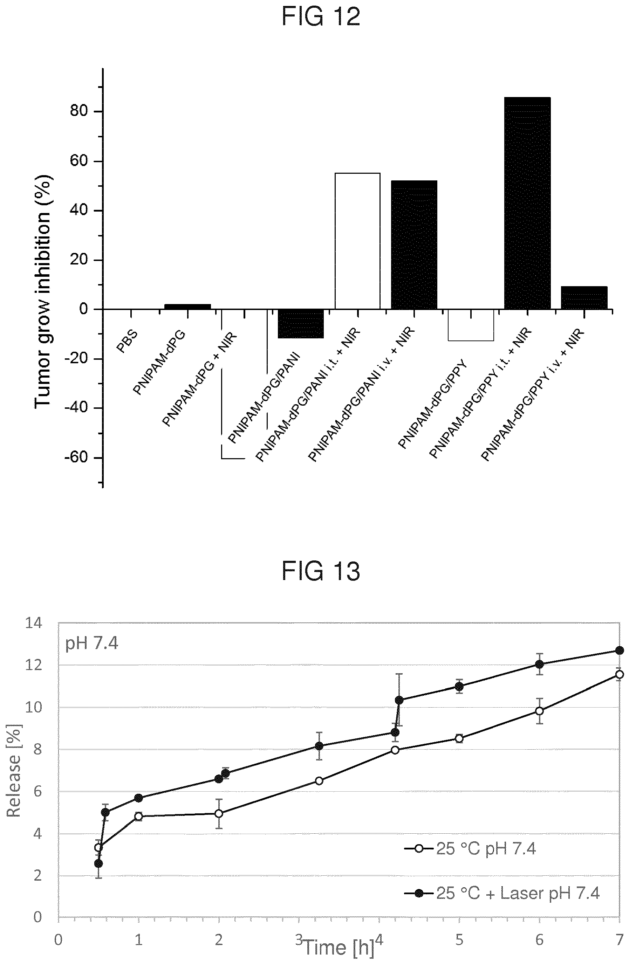

The therapeutic effect *in vivo* was also studied. A2708 tumor transplants were obtained from tumors, cut into small pieces (-2x2x2 mm) and transplanted subcutaneously (s.c.) into female nude mice (Taconic) at day 0. Mice were classified at day 14 into 10 groups with 5 mice each. Mean tumor volume at this time was 0.086 - 0.088 cm³/group. Mice were intratumorally (i.t.) or intravenously (i.v.) injected with PNIPAM-dPG/PANI or PNIPAM-dPG/PPY using PBS and PNIPAM-dPG as controls. At indicated times, mice were narcotized and tumors exposed to NIR laser light at distance of about 5 cm for 5 min at a maximum radiation power of 500 mW. Temperature profiles of each mouse were taken during each exposure. Two to three times a week, body weight and tumor volume (TV) was measured. The experiment was finished at day 39. Figure 12 shows the obtained results.

As can be seen in Figure 12, there is no or only a weak inhibitory effect of the nanogel compounds without irradiation as well as with the control PNIPAM-dPG with NIR. Contrary to that, the injection of PNIPAM-dPG/PANI followed by treatment with NIR light resulted in a tumor growth inhibition by 58% (tumor volume change - treated to control (T/C)=42%). That was still improved if mice were treated i.t. with PNIPAM-dPG/PPY and immediately exposed to NIR laser light. In this way the best inhibition of 86% (T/C= 14%) was obtained.

Moreover, it is possible to encapsulate anticancer drugs inside the nanogel compounds in order to obtain a synergetic therapy combining photothermal with chemotherapy. To investigate the potential of the described nanogel compounds as NIR-sensitive drug delivery system, the encapsulation of DOXO and the release behavior dependent on temperature and NIR irradiation was studied.

The encapsulation results dependent on the DOXO feed for PNIPAM-dPG/PANI are given in Table 2. The higher the initial feed of DOXO, the higher the loading capacity of PNIPAM-dPG/PANI. Specifically, the loading capacity (LC) increases from 0.9% at 2.8 wt% DOXO feed to 13.2% at a DOXO feed of 53 wt%. The comparison of the nanogel compounds with bare PNIPAM-dPG shows almost a doubling of DOXO loading inside the NGs at 50 wt% DOXO feed. This indicates a strong impact of PANI on the encapsulation process since conjugated polymers can stabilize DOXO inside the nanogel compound by π-π-stacking.

**Table 2: Loading capacity (LC, in %) and loading efficiency (LE, in %) of DOXO in PNIPAM-dPG and PNIPAM-dPG/PANI dependent on initial DOXO feed per mg of nanogel compound [wt%].**

| | | **PNIPAM-dPG/PANI** | | | **PNIPAM-dPG** |
|---|---|---|---|---|---|
| **DOXO feed (wt%)** | 2.8 | 4.7 | 9.3 | 53 | 50 |
| **%LC** | 0.9 | 2.1 | 3.1 | 13.2 | 6.5 |
| **%LE** | 34 | 46 | 33 | 25 | 13 |

To demonstrate the ability for NIR-triggered drug release from PNIPAM-dPG/PANI, release behavior upon NIR laser irradiation was investigated. Therefore, the PNIPAM-dPG/PANI with encapsulated DOXO was exposed to NIR light before a dialysis was started and after 2 and 4 h during dialysis by placing the dialysis tube in front of a NIR-Laser (λ = 785 nm, 500 mW) for 5 min.

The results are presented in Figure 13. By comparing with the release without laser irradiation it can be clearly seen that an increase of the free DOXO could be achieved upon NIR irradiation. An abrupt rise of DOXO release directly after the irradiation is visible which can be correlated to a NIR-induced heat production by PANI leading to shrinkage of the nanogel compound.

By combining the controlled delivery of DOXO, triggered by NIR, with the photothermal ablation of the cancer cells it is expected to see a synergistic tumor growth inhibition both *in vitro* and *in vivo.* After ablation of the cancer cells by NIR absorption, the nanogel compounds will slowly continue releasing DOXO, thus inhibiting the tumor growth.

In a further experiment, PNIPAM-dPG/PANI and PNIPAM-dPG/PPY were loaded with DOXO using the thermoresponsive properties of the nanogels. The loading capacity was found to be 13-14%, which is about twice the amount that can be loaded to non-semiinterpenetrated PNIPAM-dPG NGs.

When incubated with HeLa cells at 0.1 mg/ml overnight, PNIPAM-dPG/PANI-DOX and PNIPAM-dPG/PPY-DOX were uptaken, causing a dark color in the cells. Cells were washed and irradiated as a cluster of 1 million cells with a NIR laser for 5 minutes. During irradiation, the temperature was increased by about 42 °C for PNIPAM-dPG/PANI-DOX and by about 60 °C for PNIPAM-dPG/PPY-DOX.

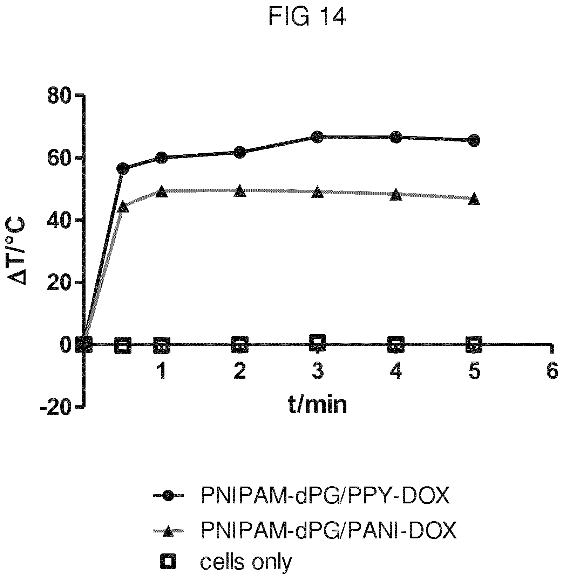

Figure 14 shows this temperature change of the examined cell cluster. Non-loaded cells do not show any temperature increase due to NIR irradiation.

To prove that this temperature increase will cause cell ablation in addition to the antiproliferative effect of DOXO, cells were reseeded after treatment and their viabilities were determined using MTT assay after 24h.

For PNIPAM-dPG/PANI-DOXO, cell viability was reduced to about 50% in non-irradiated cells, showing the cytotoxic effect of the drug alone. However, the cell viability was reduced to almost zero for the irradiated cells, showing the synergistic and combinatory effect of the two treatments (DOXO plus NIR irradiation). Similar results were obtained for PNIPAM-dPG/PPY-DOXO, with a less pronounced effect of DOXO alone, indicating a different drug release kinetics.

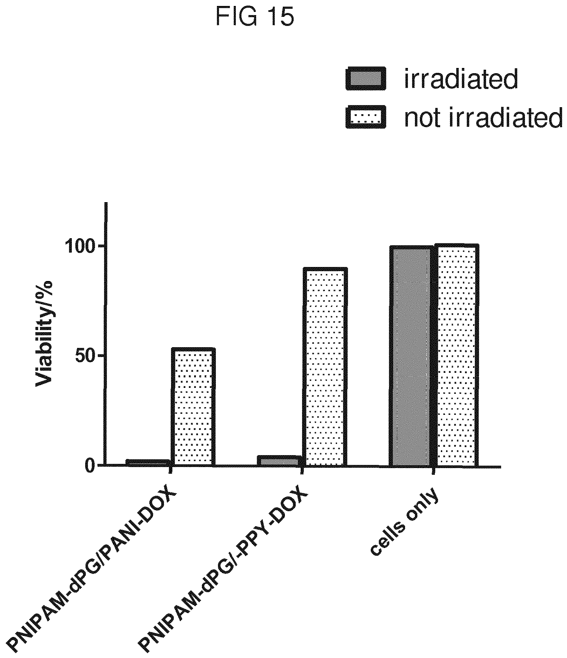

The according results are shown in Figure 15.

PNIPAM-dPG/PPY nanogels were also loaded with another drug, namely the folate analogue Methotrexate (MTX), using the thermoresponsive properties of the nanogel. The loading capacity was found to be about 18%.

The drug release profiles show that MTX release was strongly enhanced upon NIR laser irradiation. In contrast, if a small temperature trigger was provided leading to a temperature increase above the transition temperature of the nanogels, MTX release could not be increased significantly. This clearly shows that NIR treatment leads to a much more prominent temperature increase resulting in a stronger destabilization of the nanogels than a simple temperature increase above the transition temperature of the nanogels.

In summary, the inventors have formulated NIR sensitive organic nanoparticles based on PNIPAM-dPG/PANI or PNIPAM-dPG/PPY as a novel photothermal agent and used for highly effective *in vitro* hyperthermia treatment of cancer cells. By semi interpenetrating conjugated polymers in a thermoresponsive polymeric nanogel matrix component, a nanocomposite (nanogel compound) with excellent compatibility in physiological environments was obtained. This nanogel compound provides the opportunity to load the nanogel compound with drugs, such as anticancer drugs, as well as to attach targeting moieties to the nanogel matrix component, such as dPG.

Thus, these nanogel compounds are suited tools for cancer therapy. Moreover, the studies *in vivo* showed that mice tolerated up to 100 mg/kg of PNIPAM-dPG/PANI or PNIPAM-dPG/PPY, given at 5 consecutive days (accumulated dose: 500 mg kg⁻¹) without any significant sign of toxicity. And moreover, the therapeutic effect study indicated that the combination of systemic treatment with PNIPAM-dPG/PANI or PNIPAM-dPG/PPY and NIR exposure results in a higher sensitivity of the tumors for treatment with anticancer drugs if compared to the controls alone or with NIR.

Furthermore, the possibility of encapsulation and controlled slow release of DOXO after NIR irradiation was demonstrated. Combining these features, the described nanogel compounds can be applied in a combinatorial chemo- and photothermal therapy for anticancer treatment. Thus, suited applications of the described nanogel compounds are hyperthermia for cancer therapy, drug delivery triggered by NIR and combined chemo and photothermal cancer therapy.

### List of references cited in the preceding sections

1. Fleige, E.; Quadir, M. A.; Haag, R. Advanced Drug Delivery Reviews 2012, 64 (9), 866-884.
2. Ganta, S.; Devalapally, H.; Shahiwala, A.; Amiji, M. Journal of Controlled Release 2008, 126 (3), 187-204.
3. (a) Molina, M. A.; Rivarola, C. R.; Miras, M. C.; Lescano, D.; Barbero, C. A. Nanotechnology 2011, 22 (24); (b) Kawano, T.; Niidome, Y.; Mori, T.; Katayama, Y.; Niidome, T. Bioconjugate Chem. 2009, 20 (2), 209-212.
4. (a) Timko, B. P.; Dvir, T.; Kohane, D. S. Advanced Materials 2010, 22 (44), 4925-4943; (b) Yang, J.; Choi, J.; Bang, D.; Kim, E.; Lim, E. K.; Park, H.; Suh, J. S.; Lee, K.; Yoo, K. H.; Kim, E. K.; Huh, Y. M.; Haam, S. Angew. Chem. Int. Ed. Engl. 2011, 50 (2), 441-444.
5. Singh, R.; Torti, S. V. Adv. Drug Deliv. Rev. 2013, 65 (15), 2045-2060.
6. Kumar, C. S.; Mohammad, F. Adv. Drug Deliv. Rev. 2011, 63 (9), 789-808.
7. (a) Choi J, Y. J., Jang E, Suh JS, Huh YM, Lee K, Haam S. Anticancer Agents Med Chem. 2011, 11 (10), 953-964; (b) Hong, Y.; Lee, E.; Choi, J.; Oh, S. J.; Haam, S.; Huh, Y.-M.; Yoon, D. S.; Suh, J.-S.; Yang, J. Journal of Nanomaterials 2012, 2012, 1-7; (c) Yoochan Hong, E. L., Jihye Choi, Seung Jae Oh, Seungjoo Haam, Yong-Min Huh, Dae Sung Yoon, Jin-Suck Suh, and Jaemoon Yang. Journal of Nanomaterials 2012, 2012, 1-7.
8. Yuan, H.; Fales, A. M.; Vo-Dinh, T. J. Am. Chem. Soc. 2012, 134 (28), 11358-11361.
9. Zagar, T. M.; Oleson, J. R.; Vujaskovic, Z.; Dewhirst, M. W.; Craciunescu, O. I.; Blackwell, K. L.; Prosnitz, L. R.; Jones, E. L. International Journal of Hyperthermia 2010, 26 (7), 618-624.
10. Tanaka, Y.; Yamamoto, K.; Murata, T.; Nagata, K. CancerChemother. Pharmacol. 1992, 31 (1), S111-S114.
11. Chen, W.; Bardhan, R.; Bartels, M.; Perez-Torres, C.; Pautler, R. G.; Halas, N. J.; Joshi, A. Molecular Cancer Therapeutics 2010, 9 (4), 1028-1038.
12. (a) Bajpai, A. K.; Shukla, S. K.; Bhanu, S.; Kankane, S. Progress in Polymer Science 2008, 33 (11), 1088-1118; (b) Bawa, P.; Pillay, V.; Choonara, Y. E.; du Toit, L. C. Biomed. Mater. 2009, 4 (2), 022001; (c) Cabane, E.; Zhang, X.; Langowska, K.; Palivan, C. G.; Meier, W. Biointerphases 2012, 7 (1-4), 9.
13. (a) Gao, H.; Yang, W.; Min, K.; Zha, L.; Wang, C.; Fu, S. Polymer 2005, 46 (4), 1087-1093; (b) Sahiner, N.; Alb, A. M.; Graves, R.; Mandal, T.; McPherson, G. L.; Reed, W. F.; John, V. T. Polymer 2007, 48 (3), 704-711; (c) Liu, Y.-Y.; Yu, Y.; Tian, W.; Sun, L.; Fan, X.-D. Macromol. Biosci. 2009, 9 (5), 525-534.
14. Cuggino, J. C.; Alvarez I, C. I.; Strumia, M. C.; Welker, P.; Licha, K.; Steinhilber, D.; Mutihac, R.-C.; Calderon, M. Soft Matter 2011, 7 (23), 11259-11266.
15. (a) Kim, S.; Healy, K. E. Biomacromolecules 2003, 4 (5), 1214-1223; (b) López-León, T.; Ortega-Vinuesa, J. L.; Bastos-González, D.; Elaïssari, A. The Journal of Physical Chemistry B 2006, 110 (10), 4629-4636.
16. Molina, M. A.; Rivarola, C. R.; Miras, M. C.; Lescano, D.; Barbero, C. A. Nanotechnology 2011, 22 (24), 245504.
17. Giulbudagian, M.; Asadian-Birjand, M.; Steinhilber, D.; Achazi, K.; Molina, M.; Calderon, M. Polym. Chem. 2014**.**
18. Biglione, C.; Sousa-Herves, A.; Menger, M.; Wedepohl, S.; Calderon, M.; Strumia, M. C. RSC Advances 2015**.**
19. Molina, M. A.; Rivarola, C. R.; Barbero, C. A. Eur. Polym. J. 2011, 47 (10), 1977-1984.
20. Kumar, C. S. S. R.; Mohammad, F. Adv. Drug Deliv. Rev. 2011, 63 (9), 789-808.

## Claims

1. Nanogel compound comprising
• a nanogel matrix component (2, 3) comprising a dendritic polyglycerol crosslinked by poly(N-isopropylacrylamide), and
• a first conjugation component (4) which forms a semi-interpenetrating network with the nanogel matrix component,
**characterized**
**in that** the first conjugation component (4) is 2-acrylamido-2-methyl-1-propanesulfonic acid.

2. Nanogel compound according to any of the preceding claims, **characterized in that** it comprises a second conjugation component (5) which forms an interpenetrating or semi-interpenetrating network with the nanogel matrix component (3), wherein the second conjugation component (5) is chosen from the group consisting of polyaniline, polypyrrole, poly(N-(C₁-C₁₀)alkyl aniline), poly(3,4-ethylenedioxythiophene), 2-ethylhexylcyclopentadithiophene-co-2,1,3-benzothiadiazole, 2-ethylhexylcyclopentadithiophene-co-2,1,3-benzoselenadiazole, and combinations thereof.

3. Nanogel compound according to claim 2, **characterized in that** the poly(N-(C₁-C₁₀)alkyl aniline) is poly(N-methyl aniline).

4. Nanogel compound according to claim 2, **characterized in that** the second conjugation component (5) is polyaniline or polypyrrole.

5. Nanogel compound according to any of the preceding claims for use as carrier for drug delivery to a human or animal.

6. Medicament comprising a nanogel compound according to any of claims 1 to 5 and a pharmaceutically active substance (6) bound to the nanogel compound.

7. Medicament according to claim 6, **characterized in that** the pharmaceutically active substance (6) is chosen from the group consisting of doxorubicin, paclitaxel, methotrexate, cisplatinum, 5-fluorouracil and combinations thereof.

8. Method for producing a nanogel compound according to any of claims 1 to 5, comprising the steps of
• providing a nanogel matrix component (2, 3) chosen from the group consisting of dendritic polyglycerol crosslinked by poly(N-isopropylacrylamide),
• performing an in situ radical polymerization of a first conjugation component (4) in presence of the nanogel matrix component (2, 3), the first conjugation component (4) being 2-acrylamido-2-methyl-1-propanesulfonic acid.

## Patentansprüche

1. Nanogelverbindung, umfassend
• eine Nanogelmatrixkomponente (2, 3), die ein durch Poly-N-isopropylacrylamid vernetztes dendritisches Polyglycerin umfasst, und
• eine erste Konjugationskomponente (4), die ein semi-interpenetrierendes Netzwerk mit der Nanogelmatrixkomponente bildet,
**dadurch gekennzeichnet,**
**dass** es sich bei der ersten Konjugationskomponente (4) um 2-Acrylamido-2-methyl-1-propansulfonsäure handelt.

2. Nanogelverbindung nach einem der vorhergehenden Ansprüche, **dadurch gekennzeichnet, dass** sie eine zweite Konjugationskomponente (5) umfasst, die ein interpenetrierendes oder semiinterpenetrierendes Netzwerk mit der Nanogelmatrixkomponente (3) bildet, wobei die zweite Konjugationskomponente (5) aus der Gruppe bestehend aus Polyanilin, Polypyrrol, Poly-N-(C₁-C₁₀)alkylanilin, Poly-3,4-ethylendioxy-thiophen, 2-Ethylhexylcyclopentadithiophen-co-2,1,3-benzothiadiazol, 2-Ethylhexylcyclopenta-dithiophen-co-2,1,3-benzoselenadiazol und Kombinationen davon gewählt wird.

3. Nanogelverbindung nach Anspruch 2, **dadurch gekennzeichnet, dass** es sich bei dem Poly-N-(C₁-C₁₀)alkylanilin um Poly-N-methylanilin handelt.

4. Nanogelverbindung nach Anspruch 2, **dadurch gekennzeichnet, dass** es sich bei der zweiten Konjugationskomponente (5) um Polyanilin oder Polypyrrol handelt.

5. Nanogelverbindung nach einem der vorhergehenden Ansprüche zur Verwendung als Träger zur Arzneistoffzuführung an den Menschen oder ein Tier.

6. Arzneimittel, umfassend eine Nanogelverbindung nach einem der Ansprüche 1 bis 5 und einen an die Nanogelverbindung gebundenen pharmazeutischen Wirkstoff (6) .

7. Arzneimittel nach Anspruch 6, **dadurch gekennzeichnet,** der pharmazeutische Wirkstoff (6) aus der Gruppe besetehend aus Doxorubicin, Paclitaxel, Methotrexat, Cisplatin, 5-Fluoruracil und Kombinationen davon gewählt ist.

8. Verfahren zur Herstellung einer Nanogelverbindung nach einem der Ansprüche 1 bis 5, umfassend die Schritte
• Bereitstellen einer aus der Gruppe bestehend aus durch Poly-N-isopropylacrylamid vernetztem dendritischem Polyglycerin gewählten Nanogelmatrixkomponente (2, 3),
• Durchführen einer radikalischen In-situ-Polymerisation einer ersten Konjugationskomponente (4) in Gegenwart der Nanogelmatrixkomponente (2, 3), wobei es sich bei der ersten Konjugationskomponente (4) um 2-Acrylamido-2-methyl-1-propansulfonsäure handelt.

## Revendications

1. Composé de nanogel comprenant
• un composant de matrice de nanogel (2, 3) comprenant un polyglycérol dendritique réticulé par du poly(N-isopropylacrylamide), et
• un premier composant de conjugaison (4) qui forme un réseau semi-interpénétrant avec le composant de matrice de nanogel,
**caractérisé**
**en ce que** le premier composant de conjugaison (4) étant l'acide 2-acrylamido-2-méthyl-1-propanesulfonique.

2. Composé de nanogel selon l'une quelconque des revendications précédentes, **caractérisé en ce qu'**il comprend un deuxième composant de conjugaison (5) qui forme un réseau interpénétrant ou semi-interpénétrant avec le composant de matrice de nanogel (3), le deuxième composant de conjugaison (5) étant choisi dans le groupe constitué par la polyaniline, le polypyrrole, la poly(N-(C₁-C₁₀)alkyl-aniline), le poly(3,4-éthylènedioxythiophène), le 2-éthylhexylcyclopentadithiophène-co-2,1,3-benzothiadiazole, le 2-éthylhexylcyclopentadithiophène-co-2,1,3-benzosélénadiazole, et leurs combinaisons.

3. Composé de nanogel selon la revendication 2, **caractérisé en ce que** la poly(N-(C₁-C₁₀)alkyl-aniline) est la poly(N-méthylaniline) .

4. Composé de nanogel selon la revendication 2, **caractérisé en ce que** le deuxième composant de conjugaison (5) est la polyaniline ou le polypyrrole.

5. Composé de nanogel selon l'une quelconque des revendications précédentes pour une utilisation en tant que support pour une administration de médicament à un humain ou un animal.

6. Médicament comprenant un composé de nanogel selon l'une quelconque des revendications 1 à 5 et une substance pharmaceutiquement active (6) liée au composé de nanogel.

7. Médicament selon la revendication 6, **caractérisé en ce que** la substance pharmaceutiquement active (6) est choisie dans le groupe constitué par la doxorubicine, le paclitaxel, le méthotrexate, le cisplatine, le 5-fluorouracile et leurs combinaisons.

8. Procédé pour la production d'un composé de nanogel selon l'une quelconque des revendications 1 à 5, comprenant les étapes de
• mise à disposition d'un composant de matrice de nanogel (2, 3) choisi dans le groupe constitué par le polyglycérol dendritique réticulé par le poly(N-isopropylacrylamide),
• réalisation d'une polymérisation radicalaire *in situ* d'un premier composant de conjugaison (4) en présence du composant de matrice de nanogel (2, 3), le premier composant de conjugaison (4) étant l'acide 2-acrylamido-2-méthyl-1-propanesulfonique.
